Europäisches Patentamt

European Patent Office

Office européen des brevets

(11) Publication number: **0 376 078**
A2

## (12) EUROPEAN PATENT APPLICATION

(21) Application number: 89123158.1

(51) Int. Cl.5 **A61K 7/13**

(22) Date of filing: 14.12.89

(30) Priority: 26.12.88 JP 328328/88

(43) Date of publication of application:
04.07.90 Bulletin 90/27

(84) Designated Contracting States:
AT CH DE ES FR GB LI NL

(71) Applicant: **KAO CORPORATION**
**1-14-10, Nihonbashikayaba-cho**
**Chuo-ku Tokyo(JP)**

(72) Inventor: **Kawase, Jiro**
**2-9-2-304, Yamate**
**Funabashi-shi Chiba(JP)**

(74) Representative: **Wächtershäuser, Günter, Dr.**
**Tal 29**
**D-8000 München 2(DE)**

(54) Dye composition for keratinous fiber.

(57) A dye composition for keratinous fibers comprising 2,6-diaminopyridine or its salt as a coupling substance and 2,4,5,6-tetraaminopyrimidine or its salt as a developer is disclosed. The dye composition is capable of dyeing keratinous fibers with a yellowish color having a high degree of vividness. In addition, the color produced possesses excellent light resistance, washing resistance, and wear resistance.

EP 0 376 078 A2

# DYE COMPOSITION FOR KERATINOUS FIBER

## BACKGROUND OF THE INVENTION

### Field of the Invention:

This invention relates to a dye composition for keratinous fibers, and, more particularly, to a dye composition for keratinous fibers which is capable of dyeing keratinous fibers to a strong tone with a yellowish color and which has excellent fastness to keratinous fibers.

### Description of the Background Arts:

Oxidizing dyes, in which a developer and a coupling agent are employed in combination, have been widely used for dyeing keratinous fibers such as hair or the like. These oxidizing dyes are used because oxidizing coloring substances produced by the oxidizing-coupling reaction of a developer and a coupling agent in the dyes strongly dye keratinous fibers such as hair or the like. Paraphenylenediamine derivatives, p-aminophenol derivatives, diaminopyridine derivatives, 4-aminopyrazolone derivatives, hetero-cyclic pyrazolone, and the like are used as the developer. As the coupling agents, m-phenylenediamine derivatives, phenol derivatives, m-aminophenol derivatives, pyrazolone derivatives, and the like are used.

These conventional oxidizing dyes have defects in their performance which are yet to be satisfied in terms of saturation or vividness of colors, dyeing capability, and fastness.

In view of this situation, the present inventors have undertaken extensive studies relating to the combination of a coupling agent and a developer in order to overcome the above problems in oxidizing dyes, and as a result, found that use of a specific diaminopyridine derivative as a coupling agent together with a specific tetraaminopyrimidine derivative as a developer for dyeing keratinous fibers provides a high degree of saturation and vividness of colors as well as good fastness. This finding has led to the completion of this invention.

## SUMMARY OF THE INVENTION

Accordingly, an object of this invention is to provide a dye composition for keratinous fibers comprising 2,6-diaminopyridine or a salt thereof as a coupling substance and 2,4,5,6-tetraaminopyrimidine or a salt thereof as a developer.

## DETAILED DESCRIPTION OF THE INVENTION AND PREFERRED EMBODIMENTS

Given as preferable examples of salts of 2,6-diaminopyridine, a coupling substance, and salts of 2,4,5,6-tetraaminopyrimidine, a developer, are their salts of inorganic acids such as hydrochloric acid, sulfuric acid, phosphoric acid, or the like; their salts of organic acids such as carboxylic acids having a linear or branched alkyl group of a $C_{1-20}$ carbon atom content, hydroxy carboxylic acids, polyhydroxy carboxylic acids, sulfonic acids, or the like. Among them, the salts of hydrochloric acid, sulfuric acid, phosphoric acid, acetic acid, propionic acid, lactic acid, citric acid, are desirable.

The desirable proportion of 2,6-diaminopyridine or its salt, a coupling component, and 2,4,5,6-tetraaminopyrimidine or its salt, a developing component, to be formulated in the dye composition of the present invention (hereinafter abbreviated as "dye composition", is approximately in the range of 1:0.8 to 1:1.5 in molar ratio. Excessive use of one component to the other is allowable in this range. One type of developer and coupling agent can be employed either independently or in combination with one or more other types of developers or coupling agents.

In addition to the above-mentioned developers or coupling agents, any known developers, conventional direct dyes, or the like compounds can be formulated to the composition of this invention, if necessary for producing a desired color.

Examples of these coupling agents are α-naphthol, o-cresol, m-cresol, 2,6-dimethylphenol, 2,5-dimethylphenol, 3,4-dimethylphenol, 3,5-dimethylphenol, benzcatechin, pyrogallol, 1,5-dihydroxynaphthalene, 1,7-dihydroxynaphthalene, 5-amino-2-methylphenol, hydroquinone, 2,4-diaminoanisole, m-toluylenediamine, 4-aminophenol, resorcin, resorcinmonomethylether, m-phenylenediamine, 1-phenyl-3-methyl-5-pyrazolone, 1-phenyl-3-amino-5-pyrazolone, 1-phenyl-3,5-diketopyrazolidine, 1-methyl-7-dimethyl-amino-4-hydroxy-2-quinolone, 1-amino-3-acetyl-acetamino-4-nitrobenzole, 1-amino-3-cyanacetyl-amino-4-nitrobenzole, m-aminophenol, 4-chlororesorcin, 2-methylresorcin, 2,4-diaminophenoxyethanol, 3,5-diaminotrifluoromethylbenzene, 2,4-diamino-fluorobenzene, 3,5-diamino-fluorobenzene, 2,4-diamino-6-hydroxypyrimidine, 2,4,6-triamino-pyrimidine, 2-amino-4,6-dihydroxypyrimidine, 4-amino-2,6-dihydroxypyrimidine, 4,6-diamino-2-hydroxypyrimidine, p-nitro-o-phenylenediamine, 2-amino-5-nitrophenol, p-nitro-m-phenylenediamine, o-nitro-p-phenylenediamine, 2-amino-4-nitrophenol, and the like. Examples of the developers are compounds having one or more $NH_2$-, $NHR_1$-, or $N(R_1)_2$-groups, wherein $R_1$ represents an alkyl or hydroxyalkyl group of a $C_{1-4}$ carbon atom content, such as p-phenylenediamine, p-toluylenediamine, N-methyl-p-phenylenediamine, N,N-dimethyl-p-phenylenediamine, N,N-diethyl-2-methyl-p-phenylenediamine, N-ethyl-N-(hydroxyethyl)-p-phenylenediamine, chloro-p-phenylenediamine, N,N-bis-(2-hydroxyethyl)-p-phenylenediamine, methoxy-p-phenylenediamine, 2,6- dichloro-p-phenylenediamine, 2-chloro-6-bromo-p-phenylenediamine, 2-chloro-6-methyl-p-phenylenediamine, 6-methoxy-3-methyl-p-phenylenediamine, 2,5-diaminoanisol, N-(2-hydroxypropyl)-p-phenylenediamine, N-2-methoxyethyl-p-phenylenediamine, and the like; 2,5-diaminopyridine derivatives, N-butyl-N-sulfobutyl-p-phenylenediamine, and the like.

Hair or other materials are colored by the dye composition of this invention through an oxidizing coupling reaction of the components with the aid of oxygen in the air. Effecting the oxidizing coupling reaction with the aid of a chemical oxidizing agent, however, is more desirable. Especially preferable oxidizing agents are hydrogen peroxide, hydrogen peroxide-adduct of urea, melamine, or sodium borate, or a mixture of one of these hydrogen peroxide-adducts and potassium peroxide-disulfate, and the like.

It is usually desirable to provide the dye composition of this invention in the form of either a cream, emulsion, gel, solution, or the like. Preparing the composition in such forms can be accomplished according to the conventional methods. In this instance, in addition to the developers and coupling agents, various ingredients which are commonly used in cosmetics are formulated into the composition. Such ingredients include wetting agents (emulsifiers), solubilizing agents, viscosity increasing agents, stabilizers, tactile sense improvers, hair conditioning base components, perfumes, and the like. Wetting agents (emulsifiers) used in the composition include, for example, alkylbenzenesulfonates, fatty alcohol sulfates, alkylsulfonates, fatty acid alkanolamides, ethylene oxide adducts of fatty alcohol, and the like. Given as examples of viscosity increasing agents are methyl cellulose, starch, higher fatty alcohols, paraffin oils, fatty acids, and the like. Examples of stabilizers include reducing agents (e.g. sulfites), hydroquinone derivatives, chelating agents, and the like. Tactile sense improvers and hair conditioning base components are typified by silicones, higher alcohols, various kinds of nonionic surface active agents and cationic polymers, and the like.

The amounts of the developers plus coupling agents to be formulated into the above-mentioned form of the invented composition are 0.2 to 5% by weight, and preferably 1 to 3% by weight. The desirable amount of wetting agents (emulsifiers) and viscosity increasing agents in the composition is usually 0.5 to 30% by weight and 0.1 to 25% by weight, respectively.

When other oxidizing dyes and/or direct dyes are formulated in the dye composition of this invention, the desirable total amount of these dyes in the composition is in the range of 0.1 to 15% by weight, with especially desirable range being 1 to 8% by weight. It is desirable that the overall pH of the composition be adjusted to the range of 8 to 10.

A typical procedure for dyeing keratinous fibers using the dye composition of this invention is now illustrated. A dye fluid is first prepared by adding an oxidizing agent to the dye composition to effect oxidizing coupling of the mixture. This dye fluid is applied to the subject keratinous fibers, which are then allowed to stand for about 10 to 50 minutes, preferably 25 to 35 minutes to effect the action of the dye onto the fibers. The keratinous fibers thus sufficiently dyed are finally washed and dried. It is desirable that the temperature of the dye fluid be maintained between 15 to 40 °C.

It is possible to obtain a yellowish color with a high degree of vividness by dyeing keratinous fibers with the use of the dye composition according to the present invention. In addition, the color produced possesses excellent light resistance, washing resistance, and wear resistance.

Other features of the invention will become apparent in the course of the following description of the exemplary embodiments which are given for illustration of the invention and which are not intended to be limiting thereof.

3

EXAMPLES

Example 1

Added to 100 g of the base components listed below were 0.01 mol of a developer listed in Table 1 and 0.01 mol of a coupling agent listed in Table 2 and mixed. The mixture was then adjusted to pH 9.5 with ammonia to produce a dye composition of this invention.

To 100 g of the dye composition of this invention an equivalent weight of 6% aqueous hydrogen peroxide solution was added to prepare a dye solution. This dye solution was applied to goat hair, which was left at 30°C for 30 minutes. The hair was subsequently washed with a normal shampoo and dried. Table 5 shows the observed color tone and chroma of the dyed hair. The chroma of the composition was evaluated according to the standard shown in Table 3.

In addition, the fastness of the dye composition against shampoos was determined by using the dyed hair obtained above. The hair was washed with the shampoo and dried in the atmosphere. After this procedure was repeated ten times, the degree of color fading was measured using a Minolta colorimeter CR-100 as ΔE which was the color difference before and after the treatment. The degree of the color fastness is shown by the rank explained in Table 4 as a matter of convenience to correspond to the evaluation by naked eye. The results are also shown in Table 5.

| Base components: | |
|---|---|
| oleic acid | 10 % |
| diethanolamide oleate | 8 % |
| oleyl alcohol | 2 % |
| polyoxyethyleneoctyldodecylether (Average EO mols: 20) | 10 % |
| ethanol | 15 % |
| propylene glycol | 10 % |
| ammonium chloride | 3 % |
| 25% aqueous ammonium | 7 % |
| water | 35 % |

**TABLE 1** Developers

P 1 : 2,4,5,6-tetraaminopyrimidine
P 2 : p-aminophenol

**TABLE 2** Coupling Agents

C 1 : 2,6-diaminopyridine
C 2 : p-amino-o-cresol
C 3 : resorcin
C 4 : 1-naphthol
C 5 : 1,5-dihydroxynaphthalene
C 6 : 1,7-dihydroxynaphthalene
C 7 : 2,4-dihydroxypyridine
C 8 : 4-methyl-2,4-dihydroxypyridine

Table 3

| Evaluation Standard of Chroma * | |
|---|---|
| Δλ ( ε/2 ) | Evaluation |
| < 70 nm | A |
| 70 - 90 nm | B |
| 90 - 120 nm | C |
| > 120 nm | D |

* The chroma of a dyed hair can be usually evaluated by reflected spectrum. The narrower the width of the spectrum, the higher the chroma value. The value of chroma is conveniently expressed by Δλ (a long wave part of h lf band width) at ε/2 (half of maximum molecular absorption coefficent) of reflected spectrum.

Table 4

| Evaluation Standard of Fastness against a Shampoo | |
|---|---|
| Color fading (ΔE) | Evaluation |
| < 2.0 | A |
| 2.0 - 3.0 | B |
| 3.0 - 5.0 | C |

Table 5

| | Developer | coupling agent | Dyed hair | | |
|---|---|---|---|---|---|
| | | | Color tone | Chroma | Fastness against shampoo |
| Inventive Composition | $P_1$ | $C_1$ | Golden yellow | A | A |
| Comparative Composition | | | | | |
| No. 1 | $P_2$ | $C_2$ | Orange yellow | C | C |
| No. 2 | $P_2$ | $C_3$ | Yellowish brown | D | C |
| No. 3 | $P_1$ | $C_4$ | Yellowish brown | D | B |
| No. 4 | $P_1$ | $C_5$ | Olive-brown | D | B |
| No. 5 | $P_1$ | $C_6$ | Olive-brown | D | B |
| No. 6 | $P_1$ | $C_7$ | Grayish brown | C | B |
| No. 7 | $P_1$ | $C_8$ | Yellow | B | B |

Example 2

Added to 100 g of the base components used in Example 1 was an oxidized dye shown in Table 6 in the amount indicated in Table 6 and mixed. The mixture was processed according to the same method as in Example 1 to produce a dye composition. The dye composition was applied to goat hair. A blond color with a more excellent color tone and fastness than those of conventional compositions was obtained. The results are shown in Table 6. The fastness against shampoo was evaluated according to the same standard as in Example 1.

Table 6

|  | Inventive Composition | | | |
|---|---|---|---|---|
| Oxidizing Dye | No.1 | No.2 | No.3 | No.4 |
| 2,4,5,6-tetraaminopyrimidine sulfate | 0.12 | 0.12 | 0.24 | 0.24 |
| 2,6-diaminopyridine | 0.05 | 0.05 | 0.11 | 0.11 |
| toluene-2,5-diamine | 0.12 | 0.25 | 0.60 | 0.75 |
| o-aminophenol | 0.013 | 0.03 | - | 0.23 |
| m-aminophenol | 0.005 | 0.01 | - | 0.23 |
| p-aminophenol | 0.02 | 0.04 | 0.023 | 0.23 |
| p-amino-o-cresol | 0.005 | 0.01 | - | 0.04 |
| 1-naphthol | - | - | 0.012 | - |
| Color tone | Super blond | Light blond | Medium blond | Dark blond |
| Fastness against shampoo | A | A | A | A |

Example 3

A dye composition was prepared using an intermediate of a dye together with a direct dye according to the same procedures as in Example 2 and applied to goat hair. A golden blond-copper color with a more excellent color tone and fastness than those of conventional compositions was obtained. The results are shown in Table 7. The fastness against shampoo was evaluated according to the same standard as in Example 1.

6

Table 7

|  | Inventive Composition | |
| --- | --- | --- |
| Oxidizing Dye | No.1 | No.2 |
| 2,4,5,6-tetraaminopyrimidine sulfate | 0.12 | 0.12 |
| 2,6-diaminopyridine | 0.05 | 0.05 |
| toluene-2,5-diamine | 0.20 | 0.25 |
| o-aminophenol | 0.03 | 0.03 |
| m-aminophenol | 0.01 | 0.01 |
| p-aminophenol | 0.04 | 0.05 |
| p-amino-o-cresol | 0.01 | 0.05 |
| 1-naphthol | - | - |
| resorcin | - | - |
| 4-chlororesorcin | - | - |
| 2-amino-4-nitrophenol | 0.02 | - |
| 2-nitro-p-phenylenediamine | 0.10 | 0.5 |
| Color tone | Light golden blond | Copper |
| Fastness against shampoo | A | A |

Obviously, numerous modifications and variations of the present invention are possible in light of the above teachings. It is therefore to be understood that within the scope of the appended claims, the invention may be practiced otherwise than as specifically described herein.

## Claims

1. A dye composition for keratinous fibers comprising 2,6-diaminopyridine or a salt thereof as a coupling substance and 2,4,5,6-tetraaminopyrimidine or a salt thereof as a developer.

2. The dye composition for keratinous fibers according to Claim 1, further comprising other oxidizing dyes, direct dyes, or the both.

3. The dye composition for keratinous fibers according to Claims 1 or 2, comprising an oxidizing dye consisting of 2,6-diaminopyridine or a salt thereof and 2,4,5,6-tetraaminopyrimidine or a salt thereof; and 0.1 to 15% by weight of other oxidizing dyes, direct dyes, or the both.